# EUROPEAN PATENT SPECIFICATION

(11) **EP 0 815 213 B1**
(45) Date of publication and mention of the grant of the patent: **15.06.2005**
(21) Application number: 96906549.9
(22) Date of filing: 28.02.1996
(51) Int. Cl.: C12N 15/00, C12N 5/00, C12N 15/06, A01K 67/027, C07K 16/00

(54) **AN IMMORTALIZED MAMMAL HYBRIDOMA FUSION PARTNER**
Immortalisierter Fusionspartner von Kaninchen
PARTENAIRE DE FUSION D'HYBRIDOME DE MAMMIFERE IMMORTALISE

(30) Priority: 28.02.1995 US 396383
(43) Date of publication of application: 07.01.1998
(73) Proprietor: LOYOLA UNIVERSITY OF CHICAGO, Chicago, IL 60611 (US)
(72) Inventor: KNIGHT, Katherine, L., Chicago, IL 60610 (US)
(74) Representative: Kremer, Simon Mark
(86) International application number: PCT/US1996/002264
(87) International publication number: WO 1996/027004

(56) References cited:
- EP-A- 0 200 231
- US-A- 5 073 490
- SPIEKER-POLET HELGA ET AL: "RABBIT MONOCLONAL ANTIBODIES: GENERATING A FUSION PARTNER TO PRODUCE RABBIT-RABBIT HYBRIDOMAS." PROCEEDINGS OF THE NATIONAL ACADEMY OF SCIENCES OF THE UNITED STATES, vol. 92, no. 20, September 1995 (1995-09), pages 9348-9352, XP002171934 1995 ISSN: 0027-8424
- THE EMBO JOURNAL, 1990, Vol. 9, No. 3, ROSENBAUM et al., "An E-V-Abl Transgene Elicits Plasmacytomas in Concert with an Activated Myc Gene", pages 897-905, XP002948982
- PROC. NATL. ACAD. SCI. U.S.A., May 1988, Vol. 85, KNIGHT et al., "Transgenic Rabbits with Lymphocytic Leukemia Induced by the C-Myc Oncogene Fused with the Immunoglobulin Heavy Chain Enhancer", pages 3130-3134, XP002948983
- LEUKEMIA, December 1994, Vol. 8, No. 12, SETHUPATHI et al., "Lymphoid and Non-Lymphoid Tumors in E-Myc Transgenic Rabbits", pages 2144-2155, XP002905206

## Description

### BACKGROUND OF THE INVENTION

Monoclonal antibodies are widely used in research and in the diagnosis and treatment of numerous diseases. Typically; monoclonal antibodies are produced in mice, rats or hamsters because (1) several hybridoma fusion partners have been derived from these species, and/or (2) stable heterohybridomas can be obtained by fusions between cells of various rodent species. There is, however, a critical need for hybridomas from other species that can recognize antigens and epitopes that are not recognized by mice, rat or hamster-derived reagents. Accordingly, it is an object of this invention to generate an immortalized plasmacytoma cell line, from a species such as rabbit, that can be used as a hybridoma fusion partner for generating monoclonal antibodies.

The availability of rabbit monoclonal antibodies (MAb), for example, is valuable for many reasons. First, rabbits are known to produce antibodies to many antigens that are not especially immunogenic in mice. Direct comparisons of rabbit and mouse antibodies directed against human melanoma cells have shown that rabbit and mouse antibodies recognize different epitopes. Second, rabbit antibodies are generally of higher affinity than mouse antibodies. Third, because most MAb are generated in mouse and rat, there are relatively few MAb that react with mouse or rat immunogens. This is because they do not make anti-self antibodies and because rat and mouse are phylogenetically so close that their antigens are highly similar.

Until now, a fusion partner from which rabbit MAb could be generated had not been developed because rabbit plasmacytomas were not available. Several laboratories developed mouse-rabbit heterohybridomas, but this technology has had only limited success. The earliest mouse-rabbit heterohybridomas were unstable and/or secreted only light chain fragments. Although subsequent investigators attempted to overcome this problem by using normal rabbit serum (NRS) instead of fetal calf serum (FCS) as a supplement to the culture medium, the methodology remains flawed. Because the heterohybridomas are highly unstable, they need to be subcloned every four to six weeks in order to avoid loss of antibody secretion. In our laboratory, we obtained no more than two to five heterohybridomas per fusion. In addition, such heterohybridomas are difficult to clone, and the clones are generally unstable and do not secrete antibody over a prolonged period of time.

It would be desirable to have same-species hybridomas where both the fusion partner and the cells to be fused are derived from the same species, for example, rabbit-rabbit. Such same-species hybridomas would be more stable than heterohybridomas and thereby produce long-lasting cell lines that would continue to secrete antibody over a prolonged period of time. This is because the same-species hybridomas are much less likely than heterohybridomas to delete chromosomes. Such same-species hybridomas will be useful not only for generating monoclonal antibodies, but also for studying immunoglobulin genes, including the mechanism of V, (D), J gene rearrangements, allelic exclusion and somatic diversification.

Hybridoma ,technology typically concerns the fusion of myeloma cells with lymphocytes from animals which have been immunized with a particular antigen. The resulting hybridoma cell manufactures monoclonal antibodies that are specific for a single antigenic determinant.

Some properties that flow from an ideal hybridoma cell line are (1) high cloning efficiency; (2) the ability to grow rapidly in a medium supplemented with serum; (3) no secretion of myeloma immunoglobulin (Ig); (4) stable production of large amounts of Ig after fusion; and (5) ability to grow when reinserted into the originating species.

A typical procedure for making hybridomas is as follows: (a) immunize an animal (e.g., rabbit) with a certain immunogen; (b) remove the spleen and/or lymph nodes from the immunized animal and make a cell suspension in an appropriate medium; (c) fuse the cells (e.g., spleen/lymph) with animal myeloma cells; (d) dilute and culture the mixture of unfused spleen/lymph cells, unfused myeloma cells, and fused cells in a selective medium which will not support growth of the unfused cells (myeloma, lymph, or spleen cells); (e) evaluate the supernatant in each container containing hybridoma for the presence of antibody to the immunogen; and (f) select and clone hybridomas producing the desired antibodies. Once the desired hybridoma has been selected and cloned, the resultant antibody is produced by in vitro culturing of the desired hybridoma in a suitable medium.

Alternatively, the desired hybridoma can be injected directly into a suitable host, such as a rabbit or an immunodeficient mouse (such as a SCID mouse), to yield concentrated amounts of antibody [Kennett, et al., (1981) Ed., Monoclonal Antibodies. Hybridomas: A new dimension in biological analyses, Plenum Press, New York].

### SUMMARY OF THE INVENTION

The present invention relates to a rabbit fusion partner as described in claim 1. Double transgenic rabbits that developed lymphoid cells suitable for use as a fusion partner were produced; the fusion partners were then used to make rabbit-rabbit hybridomas.

The invention further relates to producing fusion partners for the production of immunoglobulins. A method according to the invention involves using the fusion partners to produce same-species hybridomas capable of producing monoclonal antibodies.

Finally, the invention relates to transgenic rabbits carrying at least two transgenes, myc and abl. The transgenic offspring developed plasmacytomas that could be cultured and used as a fusion partner. This fusion partner was used to produce stable rabbit-rabbit hybridomas that secreted antibodies specific for the immunogen.

### DETAILED DESCRIPTION OF THE INVENTION

More particularly, the invention includes an immortalized cell line referred to herein as a hybridoma fusion partner. This fusion partner may be fused to an antibody-secreting lymphocyte, preferably a B lymphocyte, in order to produce a hybridoma capable of producing homogeneous preparations of antibodies which bind, preferably selectively, to a predetermined immunogen.

The invention further includes an immortalized lymphoid cell line used to produce the fusion partner. These lymphoid cells can be any of a number of differentiating or differentiated lymphoid cells, preferably cells which are capable of producing immunoglobulin. In a preferred embodiment, the lymphoid cells are plasmacytoma cells.

The invention includes a hybridoma, preferably a same-species hybridoma, produced by fusing the fusion partner to an antibody-secreting lymphocyte. In a preferred embodiment, a plasmacytoma-derived fusion partner is fused to a B lymphocyte.

The invention further includes transgenic rabbits used to produce the lymphoid cell line.

As used herein, a "transgene" is a DNA sequence introduced into the germline of a non-human animal by way of human intervention such as by way of the above described methods. The transgenes of the invention include DNA sequences which are capable of suppressing cognate endogenous alleles. Further, such transgenes are capable of either facilitating or inhibiting the maturation of a lymphatic cell type. Such transgenes comprise DNA sequences encoding either a lymphatic polypeptide or lymphatic polypeptide variant which may be expressed in a transgenic animal.

Transgenes are derived, for example, from DNA sequences encoding at least one polypeptide chain derived from the gene, or one polypeptide chain of an immunoglobulin (Ig) molecule.

In the case of B cells, a derivative of the heavy chain of the Ig molecule is preferred to inhibit the formation of antibody producing plasma cells derived from B cells. Generally, such transgenes are derived by deleting from the DNA sequence encoding a functionally rearranged beta chain, gamma chain or heavy chain polypeptide, all or part of the DNA sequence encoding the variable region of such molecules. Preferably all of the variable region is deleted although small segments of 5' sequences encoding an N-terminal portion of the V segment and 3' sequences encoding the C-terminal portion of the J segment may be retained in the transgene. At the very least, all or part of the variable segment should be deleted. Thus, transgenes generally comprise C regions of the beta chain, gamma chain or heavy chain polypeptides of TCR and Ig molecules respectively but may also include additional sequences encoding all or part of the J and D segments of the variable region.

Further, transgenic animals containing immunoglobulin heavy chain wherein all or part of the variable region is deleted are expected to be incapable of producing plasma cells which secrete immunoglobulins. This is because during B cell development, B cells rearrange their Ig heavy chain genes first. Once a functionally rearranged Ig heavy chain gene is generated, light chain rearrangement starts. This eventually will result in the production of complete IgM molecules containing two heavy and two light chains. Such IgMs are not secreted since their C regions contain a membrane anchoring domain. During further development, the B cells switch the use of the constant region to other constant regions that do not encode a transmembrane domain, e.g., IgG. When this switch occurs, they become plasma cells which secrete large amounts of specific immunoglobulin. In order to develop into plasma cells, the IgM producing B cells must interact with other cells of the immune system via the IgM located on the B cell surface. Since the heavy chain of the IgM variant contains the deletion of all or part of the variable region, transgenic non-human animals containing a transgene encoding such a deleted heavy chain should not be able to produce B cells which can interact with the immune system to form the mature plasma cell type.

As used herein, an immunogen is an antigen or other substance that can elicit in a vertebrate host the formation of an antibody, a specific antibody, or the generation of a specific population of lymphocytes that bind to or react with the substance.

As used herein, expression cassette refers to a sequence of DNA having at least one gene and the sequences necessary for the gene to be expressed in a pre-selected host cell. For example, the gene may be an oncogene, such as myc and/or abl. The sequences necessary for expression include, but are not limited to, a transcription initiation region (including the wild-type sequences for replication), one or more promoters, one or more inducers, one or more enhancers, one or more restriction sites (e.g. polylinker), and a poly(A) addition signal. The expression cassette may also include one or more selectable markers, such as a drug resistance gene, or a screening marker, such as an HGPRT gene.

The DNA sequences from which the transgene is derived are preferably obtained from the functionally rearranged genome of the same species of animal into which the transgene will be introduced (i.e. rabbits). The invention, however, is not limited to transgenes derived from the same species of animal used to form transgenic animals. Other examples of heterologous transgenic expression include the heterologous regulatory and structural sequences disclosed in EPO Publication No. 0247494 and PCT Publication No. W088/00239. Thus, DNA sequences from species different from that of the transgenic animal ("heterologous DNA") may be used to form the transgenic animals of the present invention. Such heterologous sequences include regulatory and secretory sequences as well as structural DNA sequences which when modified encode heterologous lymphatic polypeptide variants capable of inhibiting the formation of a mature lymphocytic cell type. The only limitation on the use of such heterologous sequences is functional. The heterologous regulatory sequences must be utilized by the transgenic animal to efficiently express sufficient amounts of the lymphatic polypeptide variant such that it is able to inhibit the formation of a mature lymphocytic cell type. Further, the heterologous lymphatic polypeptide variant when properly expressed in the transgenic animal must be capable of producing the desired depletion of a lymphocytic cell type. Further, it should be possible to mix homologous and heterologous DNA sequences (e.g., homologous regulators with heterologous structural genes and vice wersa) to produce functional transgenes which may be used to practice the invention. Alternatively, heterologous DNA sequences encoding lymphatic polypeptide variant must be capable of inhibiting the expression of functional lymphatic polypeptide required for the maturation of a lymphocytic cell type by disrupting the expression of cognate endogenous alleles.

As used herein, culture medium or tissue culture refers to a medium that supports the growth of cells. Preparation of culture media is well known by those skilled in the art. Typical ingredients include, but are not limited to, a carbon source, a source of inorganic ammonia (or ammonium ion), a source of phosphate, one or more hormones or growth regulators, one or more vitamins, one or more salts, one or more amino acids, and optionally, other nutrient sources (e.g., glucose or whole serum). Specific examples of culture media in accordance with the invention are described in more detail below. The pH of the culture medium is between 4.5 and 8.5 and the cells are incubated at about 37°C.

In a preferred embodiment of the invention, the fusion partner may be derived from cells obtained from at least one transgenic rabbit carrying myc and abl transgenes. Progeny of these rabbits develop lymphoid tumors, such as plasmacytomas, which may be harvested and developed into a good fusion partner. Fusing these plasmacytoma-derived fusion partners with spleen cells of immunized rabbits results in stable hybridomas that secrete antibodies specific for the immunogen. The hybridomas can be cloned, propagated in culture or in immunodeficient mice such as nude mice, and frozen without change in their ability to secrete specific monoclonal antibodies.

Generally, an antibody comprises two different polypeptides. The shorter polypeptide functions as the light chains of the antibody, and the longer polypeptide functions as the heavy chains of the antibody. As used herein, antibody is given a functional definition, i.e., any molecule, whether naturally-occurring, artificially induced, or recombinant, which has specific immunoreactive activity. Normally, as used herein, an antibody will include at least one variable region from a heavy or light chain.

Accordingly, a fragment of a naturally occurring or recombinant antibody molecule is encompassed within the meaning of the term "antibody". As used herein, an antibody includes a Fab protein, a F(ab')₂ protein, or an Fv or Hv protein that exhibits immunoreactive activity.

Producing the lymphoid tumor begins with selecting the desired exogenous gene and the sequences necessary for expressing that gene. One skilled in the art knows that cancerous growth or tumors produced by certain oncogenes stimulate the production of a class of lymphoid cells, such as B lineage cells, that have various intermediate and mature phenotypes. Any lymphoid cell that is capable of producing immunoglobulin may be used in accordance with the invention. In a preferred embodiment of the invention, the lymphoid cells are terminally differentiated B lymphocytes. These lymphoid cells are plasmacytomas, cells that are typically identified by a characteristic staining pattern with Wright-Giemsa stain and by chromosomal rearrangements of immunoglobulin. V-(D) and J gene rearrangements are detectable by using a Southern blot analysis.

In accordance with the invention, the plasmacytomas develop in response to the expression of oncogenes. More than 20 oncogenes have now been isolated. The oncogenes used in the present invention are myc and abl.

It is well known in the art how to isolate and/or obtain an oncogene, and it is equally well known how to construct an expression vector or cassette having an oncogene capable of expression in a suitable cell type. For example, it is known in the art to express a myc gene by using an immunoglobulin heavy chain enhancer, such as the murine system described in Adams, et al., Nature, 318: 533-38 (1985), incorporated herein by reference. Such a system results in the early and strong expression of the myc gene.

In accordance with the invention, as will be explained below, it may be desirable to induce expression of an oncogene more slowly and/or with less strength. In accordance with a preferred embodiment of the invention, the myc gene may be expressed under the control of an immunoglobulin light chain enhancer (EK); and the abl gene may be expressed under the control of an immunoglobulin heavy chain enhancer (Eµ). When these transgenes are introduced into the germ line of a rabbit, expression of the transgene may result in tumor growth. An activated oncogene sequence, as the term is used herein, means an oncogene which, when incorporated into the genome of the animal, increases the probability of the development of neoplasms (particularly malignant tumors) in the animal. As noted above, plasmacytomas form as a result of the tumor growth, and, in accordance with one aspect of this invention, these plasmacytomas may be isolated from transgenic rabbits.

In general, the invention features a transgenic rabbit for the production of the lymphoid cells. Any method for producing the transgenic cells and/or rabbits may be used. For example, U.S. Patent 5,087,571 discloses a method for producing a transgenic animal having an activated oncogene. Generally, the transgenic animal has germ cells and somatic cells that contain at least two gene sequences, preferably oncogene sequences, introduced into the animal or an ancestor of the animal. Typically, the oncogene expression cassette is introduced into the animal at an embryonic stage, preferably the one-cell or fertilized oocyte state, and generally not later than about the eight-cell state.

DNA can be introduced into the germ-line of the animal using different methods, e.g., introducing the genetic material into embryonic stem (ES) cells by transfection, retroviral infection, or electroporation. The most important advantage for gene transfer into animals is that cells carrying the transgene can be selected for before being injected into a blastocyst. For example, ES cells were infected with retroviral vectors, or transfected with plasmids, carrying the neo gene. This gene confers resistance to the antibiotic G418. Only ES cells that have taken up the neo gene grow in medium containing G418, and these G418-resistant cells were introduced into mouse blastocysts. Not only did the resulting animals have neo integrated into their genomes, as shown by Southern blotting, but also the gene was transmitted to the offspring, and cell lines from the F2 generation were G418-resistant. Because ES cells can be manipulated in vitro before injection into the embryo, geneticists can use homologous recombination to produce transgenic animals with mutations, specific genes or to replace a mutant gene with the normal equivalent.

The "transgenic rabbits" of the invention may be produced by introducing "transgenes" into the germline of the animal. Embryonal target cells at various developmental stages can be used to introduce transgenes. Different methods are used depending on the stage of development of the embryonal target cell. The zygote is the best target for microinjection. In the rabbit, the male pronucleus reaches the size of approximately 10-20 micrometers in diameter, which allows reproducible injection of 1-2 pi ofDNA solution. The use of zygotes as a target for gene transfer has a major advantage in that in most cases the injected DNA will be incorporated into the host gene before the first cleavage (Brinster et al. (1985) Proc. Natl. Acad. Sci. USA 82, 4438-4442). As a consequence, all cells of the transgenic animal will carry the incorporated transgene. This will in general also be reflected in the efficient transmission of the transgene to offspring of the founder, since theoretically 50% of the germ cells will harbor the transgene.

A preferred technique for transferring a cloned gene into an embryo includes microinjecting the cloned genes into fertilized eggs, which contain two pronuclei, one from the sperm (male), and one from the egg (female). These cells ultimately form about 2 picoliters of solution may be microinjected directly into one of the two pronuclei; the injected embryos are then transferred to the oviduct of a foster mother, and upon subsequent implantation in the uterus, many develop to term. The percentage of eggs that survive the manipulation and develop to term varies, but it is usually between 10 and 30 percent. Of the survivors, the number that have the foreign DNA integrated into their chromosomes is between a few percent and 40 percent. The introduced DNA appears to integrate randomly without preference for a particular chromosomal location, usually in a tandem array of many copies at a single locus. Animals that carry the foreign gene are referred to as transgenic, and the foreign DNA is termed a transgene.

There are several protocols for introducing an oncogene into an animal embryo so as to be chromosomally incorporated in an activated state. One method is to transfect the embryo with the gene as it occurs naturally, then selecting the transgenic animals in which the gene has integrated into the chromosome at a locus which results in activation.

Other activation methods involve modifying the oncogene or its control sequences prior to introduction into the embryo. One such method is to microinject the embryo with an already translocated oncogene. Other methods are to use an oncogene whose transcription is under the control of a synthetic host or viral activating promoter, or enhancer, or to use an oncogene activated by one or more base pair substitutions, deletions, or additions.

In accordance with an embodiment of the invention, the source of the lymphoid-derived fusion partner involves producing a transgenic animal having a first transgene, obtaining fertilized ova from the transgenic animal, then microinjecting (or otherwise inserting) the ova with a second gene. Progeny of this parent can then be screened for having both genes incorporated in the genome.

Another exemplary method includes a transgenic animal having a first transgene and a separate transgenic animal having a second transgene. Mating the first transgenic animal with the second will produce progeny which are double transgenic, i.e., having both the first transgene and the second transgene incorporated in the genome.

Yet another exemplary method includes microinjecting (or otherwise inserting) a first gene and a second gene into the fertilized ova of an animal and selecting offspring that are transgenic for both genes.

Retroviral infection can also be used to introduce a transgene into a rabbit. The developing embryo can be cultured in vitro to the blastocyst stage. During this time, the blastomeres can be targets for retroviral infection. Efficient infection of the blastomeres is obtained by enzymatic treatment to remove the zona pellucida. The viral vector system used to introduce the transgene is typically a replication-defective retrovirus carrying the transgene. Transfection is easily and efficiently obtained by culturing the blastomeres on a monolayer of virus-producing cells. Alternatively, infection can be performed at a later stage. Virus or virus-producing cells can be injected into the blastocoele. Most of the founders will be mosaic for the transgene since incorporation occurs only in a subset of the cells which formed the transgenic animal. Further, the founder may contain various retroviral insertions of the transgene at different positions in the genome which generally will segregate in the offspring. In addition, it is also possible to introduce transgenes into the germ line, albeit with low efficiency, by intrauterine retroviral infection of the midgestation embryo.

Another type of target cell for transgene introduction is the embryonic stem cell (ES). ES cells are obtained from pre-implantation embryos cultured in vitro and fused with embryos. Transgenes can be efficiently introduced into the ES cells by DNA transfection or by retrovirus-mediated transduction. Such transformed ES cells can thereafter be combined with blastocysts from a rabbit. The ES cells thereafter colonize the embryo and contribute to the germ line of the resulting chimeric animal. For review, see Jaenisch, R. (1988) Science 240, 1468-1474.

It will be recognized that the gene of interest may be introduced in combination with other cells. For example, it may be desirable to introduce hematopoietic stem cells carrying the transgene in conjunction with embryonic yolk sac, fetal liver, thymus, spleen, or lymph node tissue, fetal or adult bone marrow tissue, pancreatic tissue, appendix tissue, tonsil tissue and the like.

In a preferred embodiment, the chromosome of the transgenic rabbit includes an endogenous coding sequence (for the c-myc gene, hereinafter the myc gene, and the v-abl gene, hereinafter the abl gene), which is substantially the same as the oncogene sequence, and transcription of the oncogene sequence is under the control of a promoter/enhancer sequence different from the promoter/enhancer sequence controlling transcription of the endogenous coding sequence. The oncogene sequence can also be under the control of a synthetic promoter/enhancer sequence. In some cases, the promoter sequence controlling transcription of the oncogene sequence may be inducible.

One skilled in the art will recognize that progeny carrying the transgene may be identified by Southern blot analysis, by polymerase chain reaction (PCR), and/or by Northern blot analysis.

Once the transgenic animals have been produced and identified, the animals, or their progeny, may be allowed to grow until tumors resulting from the expression of the oncogene(s) are produced. One skilled in the art recognizes that aberrant eating patterns, loss of appetite, lethargy, aberrant growth patterns, and weight loss are external indicators that tumors are developing within the transgenic animal.

Animals having tumor growth may then be used to harvest various differentiated cells, such as lymphoid cells, monocytes, macrophages, B-cells, T-cells, neutrophils, erythrocytes, eosinophils, platelets, and the like. For example, the animal may be sacrificed, and differentiated cells populating the peripheral blood organs, spleen, pancreas, lymph nodes, tonsils, etc., blood, bone marrow, or other tissue(s) may be cultured. In accordance with the present invention, the preferred cells are lymphoid cells, typically plasmacytoma cells.

These lymphoid celts may be frozen and stored, or they may be used as the source of the fusion partner. As used herein, fusion partner refers to cells, typically immortalized cells, that have been altered to exhibit a selectable characteristic once the fusion partner has been used to produce a hybridoma. One skilled in the art will recognize and be capable of choosing any one of several selectable traits. As shown in more detail in the examples, a preferred embodiment of the invention involves irradiating the harvested plasmacytoma cells and culturing the irradiated cells in the presence of 3-azaguanine, a protocol which is known to produce and select HGPRT presence of a 3-azaguanine, a protocol which is known to produce and select HGPRT mutants (hyopxanthine guanosine phosphoribosyl transferase). These mutant cells, if not fused into a hybridoma, die when cultured in HAT medium, i.e., these cells exhibit a selectable trait. As used herein, a fusion partner is a lymphoid-derived cell that exhibits a selectable trait and is suitable for fusion with another suitable cell, resulting in a hybridoma. An HGPRT⁻ plasmacytoma cell is the preferred fusion partner.

In accordance with the invention, a suitable fusion partner may be fused to a lymphocyte, preferably a B lymphocyte, in order to produce a hybridoma. Typically, spleen cells from hyperimmunized rabbits, are cultured with the fusion partner under conditions which allow the cells to fuse. When HAT is added to the culture medium, non-fused fusion partner cells will die, thus allowing hybridomas to be isolated. More specific detail of this protocol is shown in the examples.

As is well known in the art, hyperimmunizing the animal with a preselected immunogen ultimately results in a hybridoma that produces monoclonal antibodies that specifically bind to the immunogen (or antigen). In order to enhance particular subsets of T- and/or B-cells, the host may be immunized with an antigen of interest to expand the population of T-cells and B-cells that specifically bind to the particular antigen. The host may be subject to extra immunizations to further enhance the desired population. In this manner, B-cells may be produced which are specific for the antigen, and may be used as splenocytes, lymph node lymphocytes, or other peripheral blood lymphocytes or lymphocytes of other tissue for fusion with a fusion partner according to the invention. More specific detail of an exemplary protocol is shown in the examples.

ANTIBODY CHARACTERIZATION PROCEDURES - in order to select hybridomas secreting antibodies reactive with the immunogen, culture supernatants from the hybridomas need to be evaluated. Prior to the screening hybridoma supernatants, natural immunogen (500 ng/ml) may be dispensed into a 96 well microtiter plates for overnight incubation at 37°C. After overnight incubation, plates were washed and the unbound sites on the plates were blocked with bovine serum albumin (BSA).

In order to select hybrids secreting antibodies with the desired reactivity, thousands of culture fluids were tested. To do this, 50 microliter (mu 1) of supernatant fluid was added to wells containing the immunogen. Supernatants were incubated overnight at 4°C. Next day the supernatant was removed and the wells were washed with BSA. Each well then received 50 mu 1 containing 10 ng of an anti-antibody conjugated to horseradish peroxidase (HRP) diluted in BSA phosphate buffered saline (PBS). Wells were incubated for 60 minutes at 37°C. The HRP was removed after incubation and wells were washed three times with the BSA-PBS mixture. The presence of bound HRP was determined by adding 50 mu 1 of the substrate θ-phenylene diamine (OPD) in phosphate buffer containing 0.15% hydrogen peroxide. HRP in combination with its substrate (OPD) results in a yellow-colored product. Development of the yellow product was allowed to occur at room temperature for 15 minutes. The enzymatic reaction was terminated by the addition of 50 mu 1 of 4.5M H₂SO₄. Measurement of the resultant reaction product was accomplished by determining optical density (OD) at 488 nm. Presence of yellow color in the wells indicated that the mouse antibody was present in the hybridoma supernatant. As was previously described, these hybrids were cloned, ascites produced[,] and purified antibody produced for additional characterization.

STANDARD ELISA CONDITIONS: Wells may be coated with protein (500 ng/ml) as capture reagent; 0.1 to 1000 ng of the antibody or antibody fragment in 50 microliters per well of phosphate buffered saline (PBS) containing bovine serum albumin (BSA) as a test sample; overnight incubation at 37 degree(s)C followed by BSA-PBS wash; 10 ng of goat anti-antibody conjugated to horseradish peroxidase in 50 microliters of BSA-PBS as detection reagent; 60 minute incubation at 37 degree(s) C followed by BSA-PBS wash; 50 microliters of o-phenylene diamine in PBS containing 0.15 % hydrogen peroxide as substrate; 15 minute development, 50 microliters of 4.5 M sulfuric acid as stop reagent; and determination of optical density (OD) at 488 nm.

Transgenic rabbits containing functionally rearranged immunoglobulin genes may be used in studying several aspects of immunoglobulin gene expression, e.g. tissue specific expression, the mechanism of segment rearrangement, allelic exclusion and repertoire development.

Monomeric immunoglobulins are composed of four chains. The chains of higher molecular weight are designated heavy (H) chains and those of lower molecular weight light (L) chains. Digestion of an immunoglobulin with proteolytic enzymes such as pepsin produces one F(ab)₂ molecule and small peptides. The F(ab)₂ portion is often referred to as an immunoreactive fragment. An immunoreactive fragment retains the biological activity and specificity of the parent immunoglobulin. Immunoreactive fragments will be used similarly to the parent immunoglobulin molecule. The advantage is they will reduce nonspecific background reactivity. If used in vivo, they are typically less immunogenic than the entire immunoglobulin, and quite useful for immunotherapy. (Handbook of Experimental Immunology, Vol. 1.3d Ed., Edited by M. M. Weir, Immunochemistry, Blackwell Scientific Publications).

The animals of the invention can also be used as a source of cells for cell culture. Cells from the animals may advantageously exhibit desirable properties of both normal and transferred cultured cells; i.e., they will be normal or nearly normal morphologically and physiologically but can, like cells such as NIH 3T3 cells, be cultured for long, and perhaps indefinite, periods of time. Further, where the promoter sequence controlling transcriptions of the oncogenic sequence is inducible, cell growth increases and other culture characteristics can be controlled by adding or eliminating the inducing factor.

Tissues of transgenic rabbits may be analyzed for the presence of the activated oncogenes, either by directly analyzing DNA or RNA, or by assaying the tissue for the protein expressed by the gene. Cells of tissues carrying the gene can be cultured using standard tissue culture techniques.

### EXAMPLE 1

### Establishing an Immortalized Plasmacytoma Cell Line from Transgenic Rabbits

We used the transgene (Tg) technology to generate rabbits with plasmacytomas from which we could develop a plasmacytoma cell line. Two rabbits transgenic for two different oncogenes, c-myc and v-abl, were mated and yielded offspring that developed plasmacytomas. One family ofTg rabbits that carried the myc oncogene linked to the light-chain enhancer (EK-myc) and a second family of Tg rabbits carried v-abl linked to the heavy chain enhancer (Eµ-abl). We mated EK-myc Tg rabbits with the Eµ-abl Tg rabbits and screened the offspring by Southern blot analysis for the presence of both transgenes. Several offspring that carried both EK-myc and Eµ-abl transgenes became ill between the ages of 8 and 19 months. When these rabbits were sacrificed, tumors had developed in various locations. Histologic analysis of these tumors revealed that the rabbits had developed immunoblastic lymphoma or early plasmacytoma.

We also used the Tg technology to generate rabbits with plasmacytomas by microinjecting zygotes from EK-myc rabbits with the Eµ-abl Tg. The injected embryos were then implanted in the oviduct of the rabbit and live offspring were delivered. One offspring, 240E1-1, carried both the Eµ-myc and Eµ-abl Tg and became ill at approximately ten months of age. When this rabbit was sacrificed, tumors had developed in various locations. Histologic analysis of these tumors revealed that the rabbit had developed immunoblastic lymphoma or early plasmacytoma.

To obtain rabbit plasmacytoma cell lines, cells were teased from the tumorous tissues and placed in culture in medium with 15% FCS. Stable cell lines were obtained from five of the six rabbits with plasmacytoma (300F1-2, 0022-3, 20337-7, 20337-8, 240E1-1). These cell lines all synthesized and secreted immunoglobulins.

### EXAMPLE 2

### Rabbit C-myc Gene

The rabbit c-myc gene was cloned from the rabbit recombinant phage library X314-6 as previously described (Knight et al., J. Immunol., 134:1245-1250) using as a probe a 5.5 Kb EcoRI fragment containing the v-myc gene (Vennstrom et al., J. Virol., 39:625-631, 1981). Positive clones were plaque purified and one, clone 14, was restriction mapped. The 5'-3' orientation of the clone was determined using appropriate v-myc probes and a synthetic DNA oligomer encoding 33 base pairs from a relatively conserved DNA sequence around the human and mouse c-myc TATAAT box (Bernard et al., EMBOJ., 1983,. 2:2375-2383).

### EXAMPLE 3

### Rabbit Kappa Chain Enhancer (EK-myc) DNA Construct

The EK region of rabbit DNA was cloned from the J-Ckl region of the b4 k chain locus: The EK1 fragment, a 1.1 kb PstI fragment and the proposed EK2 fragment, a 0.4 kb Bg1 II fragment (Emorine et al., 1983, Nature 304:447) were cloned 5' of c-myc into pUC18. The EK-myc construct was cleaved from the plasmid DNA as a 7.5 kb Barn HI/Hind III fragment.

### EXAMPLE 4

### Transgenic Rabbits

Adult female rabbits were obtained from Scientific Small Animals (Chicago, IL). Rabbit zygote donors were injected subcutaneously with 50 IU of pregnant mare gonadotropin (Signa, St. Louis, MO) on day four and immediately after mating, and injected intravenously with 150 IU of chorionic gonadotropin (HCG) (Sigma). Single cell zygotes were flushed from the oviducts 19 hours later. In some experiments, the pronuclei were injected, according to Hammer et al., Nature, 315:680-683, 1985) with the 7.5 kb Barn HI/Hind III DNA fragment (1 µg/ml) containing both the rabbit c-myc and kappa chain enhancer DNA segments which had been cloned previously into pUC 18. In other experiments, the pronuclei were injected with a 5 kb Eco RI fragment that contained the Eµ-abl construct (see Rosenbaum et al., The EMBO Journal, 9:897-905 1990. The injected zygotes were implanted on day 1 in the oviduct, through the fimbrial end, of a recipient rabbit made pseudopregnant on day 1 by intravenous injection of 150 IU of chorionic gonadotropin (HCG), or by mating with a sterile male.

### EXAMPLE 5

### Development of EK-myc/Eµ-abl Double Transgenic Rabbits

A family of transgenic rabbits that carried the c-myc oncogene linked to the κ-chain enhancer were produced. We now generated a second family of transgenic rabbits with the v-*abl* oncogene linked to the immunoglobulin heavy chain enhancer (Eµ) as a transgene. A total of 665 zygotes were microinjected and implanted in 31 pseudopregnant females. From 11 pregnant females we obtained 19 offspring, of which 2 carried the v-*abl* transgene.

EK-myc/Eµ-abl double transgenic rabbits were developed by two methods. In the first method, single cell zygotes of the EK-myc transgenic rabbit were collected and microinjected with the Eµ-abl DNA construct. The injected embryos were implanted into the oviduct of the rabbit and live offspring were delivered. Southern blot analysis of DNA from these rabbits showed that one, 24OE, was double transgenic, carrying both the Eₖ-myc and the E_{µ}-abl transgenes.

In the second method, rabbits from the Ek-myc family that carried the Ek-myc transgene were mated with rabbits from the Eµ-abl family that carried the Ek-abl transgene. Of the progeny, some carried both the Ek-myc and Eµ-abl transgene, as determined by Southern analysis. From four matings, 22 offspring were obtained, and of these, 5 carried both transgenes. The plasmacytomas 81E5-1 and 300F1-2 developed in offspring of transgenic rabbits developed in our laboratory.

All offspring that carried both transgenes, c*-myc* and v-*abl*, became ill between the ages of 8 and 19 months. Tumors had developed in these rabbits in various locations. Histologic analysis of these tumors revealed that the rabbits had developed immunoblastic lymphoma or early plasmacytoma.

### EXAMPLE 6

### Tissue culture

Single cell suspensions for tissue culture were prepared from spleen, mesenteric lymph nodes and bone marrow. Culture medium used throughout was enriched RPMI 1640: RPMI 1640 with the following additions: amino acids, non-essential amino acids, pyruvate, glutamine, vitamins, HEPES, gentamycin, penicillin, streptomycin, and fungizone. All components are commercially available from Gibco Laboratories, Grand Island NY, and were used at the concentrations as suggested by the manufacturer. The medium also contained 50 µM 2-mercaptoethanol. After 6-8 weeks in culture, stable cell lines were growing from these tumorous tissues.

### EXAMPLE 7

### Histology Analysis

Cells taken from tissue culture of the plasmacytoma cell lines and paraffin-embedded tissue sections of normal and plasmacytomatous rabbits were stained with Wright-Giemsa (Diff Quick, American Scientific Products, McGaw Park, IL) or hematoxylin and eosin, respectively.

Genomic DNA Analysis. Genomic DNA (10 µg), prepared by the method of Blin and Stafford (Nucleic Acid Research, 1976 3:2303-2308) were analyzed using methods described by Southern (J. Mol. Biol., 1975, 98:503-517 (DNA ³²P-labelled probes were prepared as previously described (Knight et al., 1985, supra).

ELISA and Immunofluorescence. Enzyme-linked immunoassay (ELISA) was performed in 96-well microtiter plates (Falcon 3912, Fisher) that were coated overnight with purified goat anti-rabbit L-chain antibody (1µg/ml) or with the immunogen (2 µg/ml). The following solutions were added, sequentially, for 1-2h at room temperature: first, the supernatants to be tested, then biotinylated goat anti rabbit L-chain or goat anti rabbit µ γ, or α-chain antibodies (1µg/ml). This was followed by incubation with avidin-biotin-horseradish peroxidase (HRP) complex (Vectastain ABC Kit, Vectastain, Vector Laboratories, Burlingame, CA, 94010) and finally with substrate, 2,2'-azinobis(3-ethylbenzlthiazolinesulfonic acid) (ABTS), as suggested by the manufacturer. Color development was read at 405 nm in an ELISA plate reader.

### EXAMPLE 8

### Development of a Rabbit Fusion Partner

To obtain a HAT-sensitive fusion partner, three cell lines were first x-irradiated with 200 Rad and then cultured in the presence of 8-azaguanine to develop an hypoxanthine guanosine phosphoribosyl transferase (HGPRT) mutant cell line that would die when cultured in the presence of hypoxanthanine aminopterin and thymidine (HAT). The concentration of 8-azaguanine was initially 0.2 µg/ml and was slowly increased to 20µg/ml over a 10-month period. We obtained three 8-azaguanine-resistant clones; 20337-7 after one month and 240E1-1-1 and 240E1-1-2 after 8 months in culture. Cells of those three clones were sensitive to medium containing HAT.

In preliminary fusions, we tested whether any of the HAT-sensitive plasmacytoma cell lines could be used as a fusion partner. We fused all three cell lines with spleen cells of a rabbit immunized with the human T-cell line, Jurkat, and we found that one of the three plasmacytoma cell lines, 240E1-1-2, produced hybridomas. We used this cell line for further fusions.

We determined the doubling time of the 240E1-1-2 cells to be 48-72 h (when grown in medium with 15% FCS). By staining with Wright-Giemsa (Dirt-Quick, American Scientific Products, Mc Gaw Park, Illinois), the cells had features characteristics of plasma cells, i.e., they are large cells with abundant cytoplasm, and the nuclei frequently contain "lumpy" chromatin. The cells had many vacuoles which indicates that they were proplasmocytes. We assayed for the presence of secreted and intracellular Ig by ELISA and found no Ig heavy or light chain in the supernatant or in the cell lysate of the 240E1-1-2 fusion partner. Thus, this fusion partner is unlike the original cell line 240E1-1 in that it neither produces nor secretes Ig. Such a nonsecreting fusion partner is advantageous because it allows us to detect hybridomas by assaying for secreted Ig rather than for secreted antibody with specificity for a given immunogen.

### EXAMPLE 9

### Fusion of Rabbit Plasmacytoma Cell Line with Rabbit Spleen Cells

Spleen cells (1.5-3 x 10⁸) of hyperimmunized rabbits and the fusion partner 240E-1-1-2 were fused at a ratio of 2:1 with 50% PEG 4000 (EM Science, Cherry Hill, NJ 08304) at 37°C in serum free medium. The cells were plated in 48-well microtiter plates at approximately 2 x 10⁵ spleen cells per well in medium with 15 % FCS. After 72 hours, HAT was added. Medium was changed every 5-6 days. Clones usually were observed after 2 weeks. At 3-5 weeks after the fusion, supernatants were tested for the presence of antibody specific for the immunogen either by immunofluorescence or by ELISA. Hybridomas were cloned by limited dilution in 48-well microtiter plates. As feeder cells we used the fusion partner 240E1-1-2, 5x10⁴ cells per well. These feeder cells were killed 5-6 days later by the addition of HAT.

### EXAMPLE 10

### Production of MAb

To further test whether we could obtain stable antibody producing hybridomas, we fused the newly established plasmacytoma line, 240 E1-1-2, with spleen cells from rabbits hyper-immunized with three different antigens, the human T cell line, Jurkat; ovalbumin; or mouse serum proteins, including immunoglobulins that were precipitated with 45% saturated (NH₄)₂SO₄. We chose these immunogens for the following reasons: Jurkat cells because they are a source of cell surface antigens; ovalbumin because it is a well-known immunogen for rabbit; and mouse Ig because monoclonal isotype-specific antibodies to mouse Ig would be valuable reagents. From all three fusions we obtained hybridomas that secreted MAb specific for the immunogen (Table 1). We used immunofluorescence to test the supernatant of hybridomas obtained from spleen cells of a rabbit immunized with Jurkat cells and found that 10 of 104 hybridomas secreted antibodies that bound to Jurkat cells. We used ELISA to test the supernatant from the hybridomas of the two other fusions and found that 9 hybridomas secreted antibodies specific for ovalbumin and that 43 hybridomas secreted antibodies that recognized antigens of the mouse serum proteins that were used to immunize the rabbit. We also need ELISA to test the supernatant of some of the MAb to mouse serum proteins for their ability to bind to the different mouse Ig isotypes. We found that several of the MAb recognize mouse IgG. We used immunofluorescence to test one MAb that recognizes mouse IgG2 and showed that it binds to IgG2a-expressing B lymphoma cells, A20.

The fusion efficiency for the three fusions performed was between 0.25 and 1.2 in 10⁶ cells, which is comparable to the efficiency generally obtained in mouse-mouse fusions. Of the hybridomas produced, between 9% and 24% secreted MAb that were specific for the immunogens (Table 1). Again, this percentage of hybridomas that secretes specific MAb is comparable to that obtained in mouse-mouse fusions. Using ELISA, we determined the isotype of the antibodies secreted by the hybridomas specific for mouse serum proteins and found that all 43 of them were of the IgG isotype. Of these 43 antibody-secreting clones, we subcloned 7, all of which secreted MAb. The hybridomas could be frozen and thawed without loosing their ability to secrete MAb. These data indicate that the hybridomas are stable and that frequent cloning, which has been necessary for the heterohybridomas, is not needed for the rabbit-rabbit hybridomas.

The concentration of MAb in the supernatant was determined by ELISA. It generated approximately 10 ng/ml, a low concentration that is to be expected if the fusion partner is a proplasmacyte rather than a mature plasma cell. Concentrations of MAb of 1 µg/ml could be obtained in ascites of nude mice. Both the supernatant and the ascites can be used in fluorescence labeling of cell-surface antigens. Most of all of the rabbit MAb are of the IgG isotype, and since rabbit IgG binds protein A and protein G as well as complement, these MAb will also be useful for immunoprecipitation and cytotoxicity assays.

### EXAMPLE 11

### Frequency and Stability of Hybridomas Obtained in Three Fusions of the Rabbit Fusion Partner with Spleen Cells from Hyperimmunized Rabbits

Fusions were performed using conventional methodology: spleen cells (1.5 - 3 x 10⁴) of hyperimmunized rabbits and the fusion partner 240E1-1-2 were fused at a ratio of 2:1 with 50% PEG 4000 (EM Science, Cherry Hill, NJ 08304) at 37°C in serum-free medium. The cells were plated in 48-well microtiter plates, at approximately 2 x 10⁴ spleen cells per well, in medium with 15% FCS. After 72 h, HAT was added; the medium was changed every 5-6 days. Clones usually were observed after 2 weeks. At 3-5 weeks after the fusion, supernatant were tested for the presence of antibody specific for the immunogen, either by immunofluorescence with Jurkat cells (by using FITC goat anti-rabbit L-chain antibody as secondary reagent) (fusion 1) or by ELISA (fusions 2 and 3). Hybridomas were cloned by limiting dilution in 48-well microtiter plates. For feeder cells, we used the fusion partner, 240E1-1-2, 5 x 10⁴ cells per well. These feeder cells were killed 5-6 days later by the addition of HAT.

**TABLE 1**

| Fusion No: Immunogen | Wells with Hybrids/wells plated (%) | Hybrids 10⁶ cells fused | Hybrids secreting specific Ab/total hybrids tested (%) | Hybrids yielding stable clones/total hybrids cloned (%) |
|---|---|---|---|---|
| 1: Jurkat cells | 200/400 (50) | 0.7 | 10/104(10) | not done |
| 2: Ovalbumin | 38/980* (4) | 0.25 | 9/11(9) | not done |
| 3: Mouse serum proteins | 242/980(25) | 1.2 | 43/187(24) | 7/7 (100) |

| | | | | |
|---|---|---|---|---|
| **In many wells adherent cells were growing that prevented the growth of the upcoming hybridomas. In such cases the hybridoma clones had to be removed from the adherenmt cells, and this was done only with 38 clones*. | | | | |

In the original screening, the supernatant of most wells that contained hybridomas had antibodies that appeared to be specific to the antigen. However, most wells also contained adherent cells, which seemed to support the growth of primary lymphocytes that secreted specific antibody for several weeks. Only after removing the lymphocytes from the layer of adherent cells could we identify true hybridomas. This difficulty likely explained the low number of positive wells in this fusion.

In preliminary fusion experiments we noticed excessive growth of adherent cells in several wells, which prevented the hybridomas from establishing themselves. The extent of this growth varied from one experiment to another, and it could be partially prevented if fetal calf serum (FCS) was totally or partly replaced by normal rabbit serum (NRS). However, the hybridomas appeared to grow more slowly in the absence of FCS. In one experiment, we attempted to remove the adherent cells by incubating the spleen cell suspension on plastic dishes for 6 hours at 37°C before fusing them. Although this method did not eliminate adherent cell growth, it did reduce the number of wells with adherent cells.

### EXAMPLE 12

### Immunofluorescence Labeling of Mouse B-Lymphoma Cells A20 with Monoclonal Rabbit Anti-Mouse IgG2 Antibody

A20 cells were incubated with the supernatant of a rabbit-rabbit hybridoma (fusion 3) that was shown by ELISA to recognize mouse IgG2a and IgG2b but none of the other mouse Ig isotypes. In control samples, A20 cells were incubated with the supernatant of IgG-secreting rabbit-rabbit hybridoma that recognizes an irrelevant antigen, i.e., a surface antigen of Jurkat cells (fusion 1). As secondary antibody we used FTTC-conjugated goat anti-rabbit L-chain.

To examine the utility of these MAbs in immunofluorescence experiments, we tested two of the MAbs that recognize IgG2a for binding to IgG2a-expressing B-lymphoma cells, A20, and we found that both MAbs bind to A20 cells, while a MAb that was shown by ELISA to recognize only IgG2b did not bind to the A20 cells. We conclude that the rabbit MAbs will be valuable immunofluorescent reagents.

The hybridomas have been subcloned, and they were frozen and thawed without loss in their ability to secrete MAb. These data indicate that the hybridomas are stable and that frequent cloning, which had been necessary for the heterohybridomas, is not needed for the rabbit-rabbit hybridomas.

### EXAMPLE 13

### Cloning the HPRT Gene

Animal cells with a homozygous mutation for HPRT were grown in a medium containing hypoxanthine, aminopterin, and thymidine (HAT). Only HPRT⁺ cells can grow in this medium, so the cells that survived and gave rise to colonies were HPRT⁺ revertants. In vitro translation of mRNA from such cells showed that the cells were overexpressing HPRT mRNA. This mRNA may be used to prepare a cDNA library that was differentially screened with radioactively labeled cDNAs prepared from mRNA from the revertant and HPRT cells. A single clone that did not hybridize with HPRT cell cDNA and did hybridize with HRPT⁺ revertant cell cDNA was isolated and shown to contain HPRT cDNA by in vitro translation of hybrid-selected mRNA. This animal cDNA was then used to screen a DNA library at low stringency.

### EXAMPLE 14

### Embryonic Stem Cells (ES cells) Produced from Blastocysts

Rabbits are mated, and 3 days later, blastocysts are isolated and cultured in petri dishes. The cells spread out over the surface of the dish so that the clump of cells forming the inner cell mass, and corresponding to the future embryo, can be removed. The clump of cells is dissociated into single cells using trypsin, a proteolytic enzyme. If ES cells are plated out on a plain culture-dish surface, they will differentiate into a variety of tissues, but if they are grown on a feeder layer of fibroblasts, they will continue to proliferate and can be subcultured repeatedly. A feeder layer, as used herein, refers to a monolayer of cells that has been treated so that the cells can no longer divide. They continue to metabolize, and in so doing "condition" the culture medium so that the cells seeded on top of them survive and grow better. The cells can be microinjected into a blastocyst, where they will become assimilated into the inner cell mass and take part in the formation of many tissues of the chimeric animal. It is usual to use ES cells and recipient blastocysts derived from animals with different phenotypes so that the contribution of the ES cells to the chimeric offspring can be assessed by simply looking for the phenotype.

### EXAMPLE 15

### Production of IgA-Secreting Hybridomas

Most of the hybridomas from spleen secreted IgG and none were found that secreted IgA (Table 2). Because IgA-producing hybridomas would be valuable reagents, we decided to perform fusions with cells from Peyer's patch (PP) and mesenteric lymph node (MLN) to obtain IgA-secreting hybridomas. From two separate fusions 34% of the hybridomas from MLN and 81% of the hybridomas from PP secreted IgA (Table 2). Similar results were obtained in two additional experiments, i.e., 35% of the hybridomas from MLN and 38% of the hybridomas from PP secreted IgA A high percentage of IgA-producing hybridomas from MLN, PP, and other mucosal tissues had been found by other investigators for rats. In contrast, fusion with spleen cells does not generally yield IgA-secreting hybridomas in either mice or rats, as we now report for rabbits.

**TABLE 2**

| Monoclonal antibodies produced by rabbit hybridomas obtained from fusions of 240E1-1-2 with mesenteric lymph node (MLN), Peyer's patch (PP), or spleen cells. | | | | |
|---|---|---|---|---|
| | **Number of clones (% of total clones)** | | | |
| **Cells fused** | **Total** | **IgG** | **IgM** | **IgA** |
| **Spleen*** | **25** | **25 (100)** | **0** | **0** |
| **MLN**** | **82** | **14 (17)** | **34 (41)** | **28 (34)** |
| **PP***** | **48** | **2 (4)** | **2 (4)** | **39 (81)** |

| | | | | |
|---|---|---|---|---|
| **Data from fusion 3, Table 1: 25 of 43 specific mAbs were analyzed*. | | | | |
| ***MLN cells from an unimmunized rabbit were activated by murine CD40-ligand-transfected CHO cells for 48 hours prior to fusion*. | | | | |
| ****PP cells were treated as described above for MLN cells and plated in 400 wells*. | | | | |

### DEPOSITS

The rabbit plasmacytoma cell line (designated 240E1-1), the rabbit fusion partner (designated 240E-1-2), and a hybridoma obtained from fusing the fusion partner, 240E1-1-2, with spleen cells of a hyper-immunized rabbit have been deposited with the American Type Culture Collection, 12301 Parklawn Drive, Rockville, Maryland, 20852 USA. The plasmacytoma cell line (240E1-1) received ATCC Accession No. CRL-11872; the rabbit fusion partner (designated 240E-1-2) received ATCC Accession No. HB-11870; and the hybridoma obtained by fusing fusion partner 240E1-1-2 with spleen cells received ATCC Accession No. HB-11871.

Although the present invention has been described in terms of particular preferred embodiments, it is not limited to those embedments. Alternative embodiments, examples, and modifications falling within the scope of the claims will be apparent to those skilled in the art, particularly in light of the foregoing teachings.

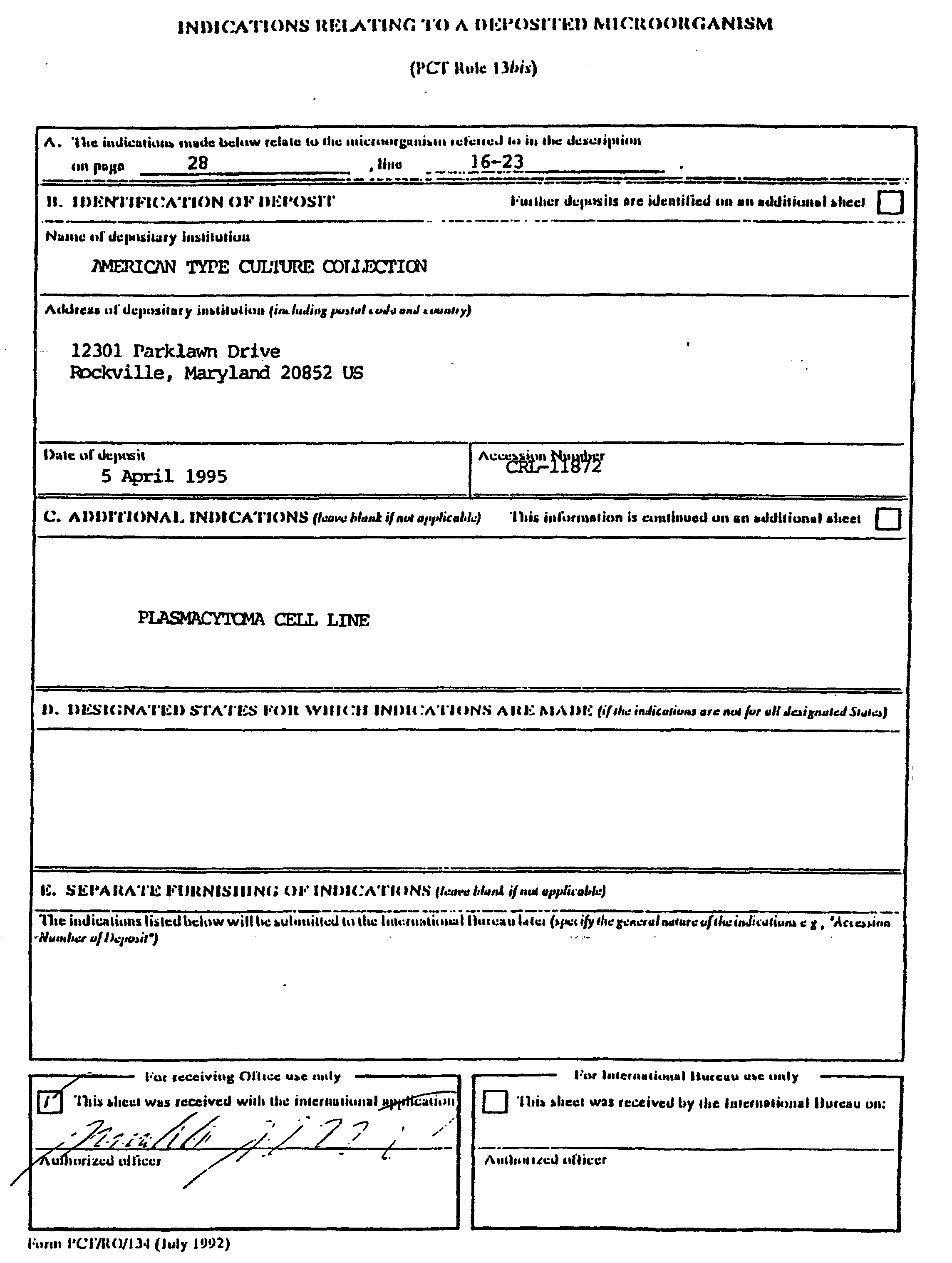
INDICATIONS RELATING TO A DEPOSITED MICROORGANISM

(PCT Rule 13bis)

A. The indications made below relate to the microorganism referred to in the description
on page 28 , line 16-23 .

B. IDENTIFICATION OF DEPOSIT | Further deposits are identified on an additional sheet [ ]

Name of depositary institution

AMERICAN TYPE CULTURE COLLECTION

Address of depositary institution (including postal code and country)

12301 Parklawn Drive
Rockville, Maryland 20852 US

Date of deposit | Accession Number
5 April 1995 | CRL-11872

C. ADDITIONAL INDICATIONS (leave blank if not applicable) | This information is continued on an additional sheet [ ]

PLASMACYTOMA CELL LINE

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e g , "Accession Number of Deposit")

For receiving Office use only | For International Bureau use only

[/] This sheet was received with the international application | [ ] This sheet was received by the International Bureau on:


Authorized officer | Authorized officer

Form PCT/RO/134 (July 1992)

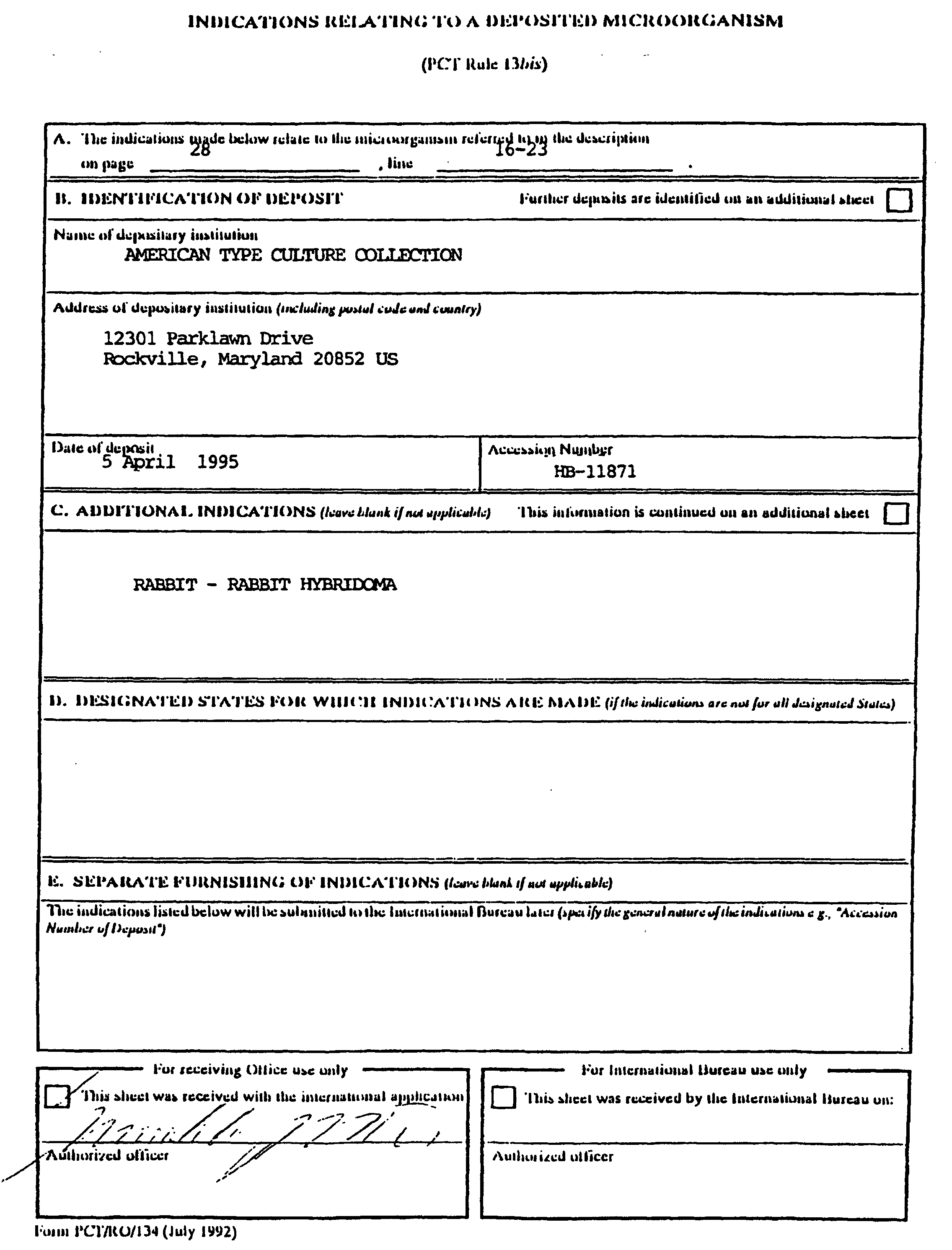
INDICATIONS RELATING TO A DEPOSITED MICROORGANISM

(PCT Rule 13bis)

A. The indications made below relate to the microorganism referred to in the description
on page 28 , line 16-23 .

B. IDENTIFICATION OF DEPOSIT | Further deposits are identified on an additional sheet [ ]

Name of depositary institution
AMERICAN TYPE CULTURE COLLECTION

Address of depositary institution (including postal code and country)
12301 Parklawn Drive
Rockville, Maryland 20852 US

Date of deposit | Accession Number
5 April 1995 | HB-11871

C. ADDITIONAL INDICATIONS (leave blank if not applicable) | This information is continued on an additional sheet [ ]

RABBIT - RABBIT HYBRIDOMA

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only

[✓] This sheet was received with the international application | [ ] This sheet was received by the International Bureau on:


Authorized officer | Authorized officer

Form PCT/RO/134 (July 1992)

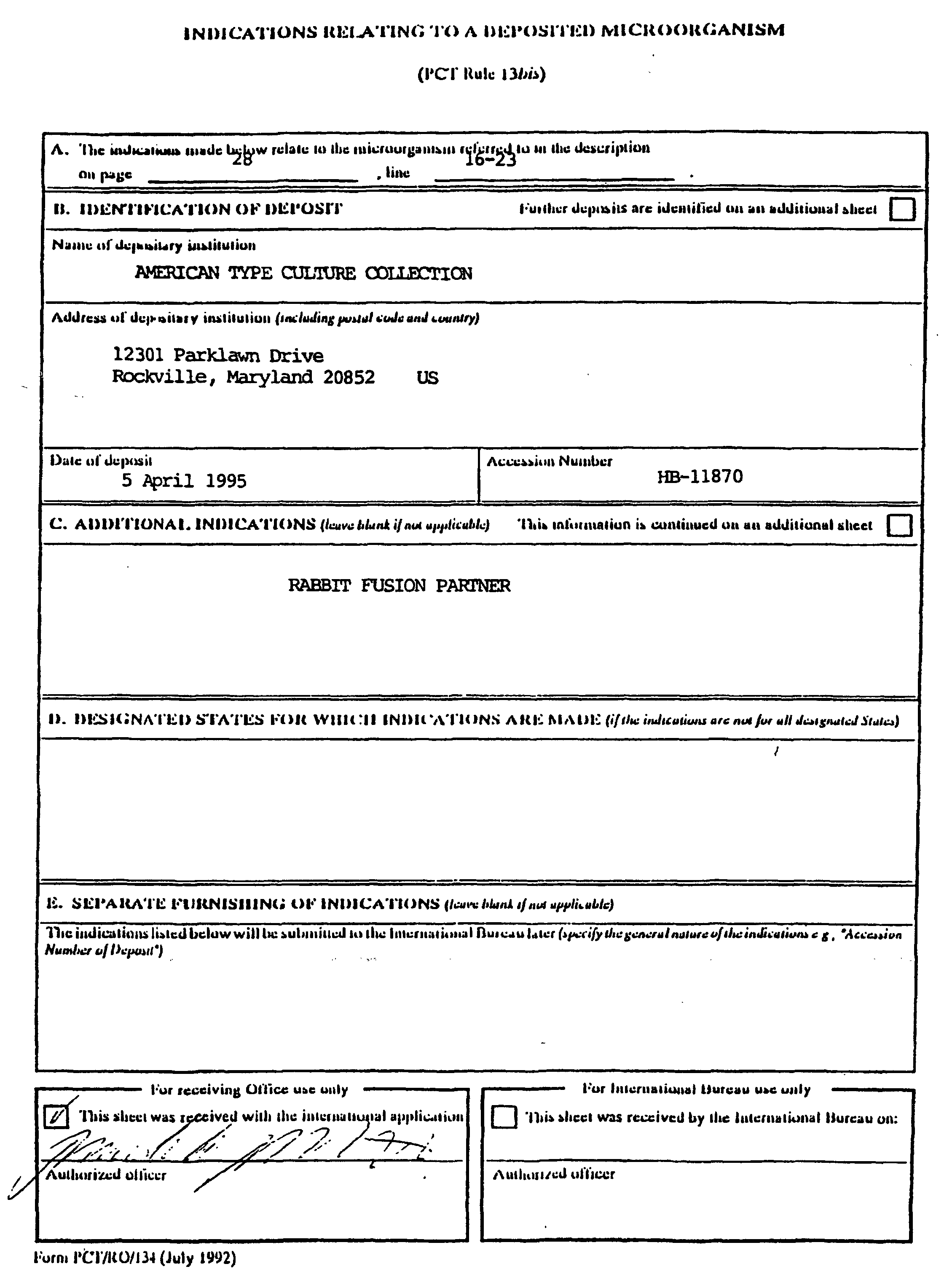
INDICATIONS RELATING TO A DEPOSITED MICROORGANISM

(PCT Rule 13bis)

A. The indications made below relate to the microorganism referred to in the description
on page 28 , line 16-23 .

B. IDENTIFICATION OF DEPOSIT | Further deposits are identified on an additional sheet ☐

Name of depositary institution
AMERICAN TYPE CULTURE COLLECTION

Address of depositary institution (including postal code and country)
12301 Parklawn Drive
Rockville, Maryland 20852 US

Date of deposit | Accession Number
5 April 1995 | HB-11870

C. ADDITIONAL INDICATIONS (leave blank if not applicable) | This information is continued on an additional sheet ☐

RABBIT FUSION PARTNER

D. DESIGNATED STATES FOR WHICH INDICATIONS ARE MADE (if the indications are not for all designated States)

E. SEPARATE FURNISHING OF INDICATIONS (leave blank if not applicable)
The indications listed below will be submitted to the International Bureau later (specify the general nature of the indications e g , "Accession Number of Deposit")

For receiving Office use only | For International Bureau use only
☑ This sheet was received with the international application | ☐ This sheet was received by the International Bureau on:
Authorized officer | Authorized officer

Form PCT/RO/134 (July 1992)

## Claims

1. A rabbit fusion partner comprising a transformed rabbit B-lymphocyte which expresses a myc oncogene and an abl oncogene and is capable of fusing with an activated B-lymphocyte, wherein fusion of the rabbit fusion partner and the activated B-lymphocyte results in a hybridoma that produces an immunoglobulin.

2. A rabbit fusion partner of claim 1, which is the rabbit fusion partner of ATCC Accession Number HB-11870.

3. A rabbit hybridoma cell line that results from the fusion of a rabbit fusion partner of claim 1 or claim 2 and an activated B-lymphocyte.

4. The rabbit hybridoma cell line of claim 3, which is the hybridoma cell line of ATCC Accession Number HB-11871.

5. The rabbit plasmacytoma cell line of ATCC Accession Number CRL-11872.

6. A fertilized rabbit egg comprising
(i) an expression cassette containing a myc oncogene and an expression cassette containing an abl oncogene, or
(ii) an expression cassette containing a myc oncogene and an abl oncogene,
wherein a transgenic rabbit developed from said egg comprises germ cells and somatic cells that contain said two oncogenes, wherein the expression of said two oncogenes together in its B-lymphocytes results in the transformation of said B-lymphocytes.

7. A transgenic rabbit whose germ cells and somatic cells comprise a myc oncogene and an abl oncogene, each operably linked to a transcription initiation region specific for expression in rabbit B-lymphocytes, wherein the expression of said two oncogenes together results in the transformation of said B-lymphocytes.

8. The rabbit fusion partner of claim 1, the fertilized rabbit egg of claim 6, or the transgenic rabbit of claim 7, wherein each said oncogene comprises the wild-type transcription initiation region for said oncogene and a lymphocyte specific enhancer.

9. The rabbit fusion partner, fertilized rabbit egg or transgenic rabbit of claim 8, wherein said oncogenes are a c-myc oncogene operably linked to an immunoglobulin light-chain enhancer, and a v-abl oncogene operably linked to an immunoglobulin light- or heavy-chain enhancer.

10. The rabbit fusion partner, fertilized rabbit egg or transgenic rabbit of claim 9, wherein said oncogenes are an E_{κ}-myc oncogene and an E_{µ}-abl oncogene.

11. A method of preparing a rabbit fusion partner comprising:
(a) culturing transformed B-lymphocytes obtained from a transgenic rabbit according to claim 7 in the presence of a selecting agent; and
(b) selecting said transformed B-lymphocytes, wherein said selecting results in B-lymphocytes that comprise a selectable trait and are suitable for use as a rabbit fusion partner.

12. The method of claim 11, wherein said selecting results in HGPRT⁻ cells.

13. A method of producing a rabbit hybridoma cell line comprising fusing the rabbit fusion partner of claim 1 or claim 2 with an activated rabbit B-lymphocyte under fusing and selective conditions to obtain a hybridoma that is capable of producing immunoglobulins.

14. A method of producing monoclonal rabbit immunoglobulins comprising producing a rabbit hybridoma cell line by the method of claim 13, and culturing said hybridoma under conditions such that said immunoglobulins are produced.

15. The method of claim 14, wherein said activated rabbit B-lymphocyte is isolated from a rabbit injected with an antigen.

16. A method of culturing a rabbit fusion partner which comprises growing the cells of a rabbit fusion partner of claim 1 or claim 2 in an enriched culture medium comprising amino acids, essential amino acids, pyruvate and glutamine.

## Patentansprüche

1. Kaninchen-Fusionspartner, umfassend einen transformierten Kaninchen-B-Lymphozyten, der ein myc-Onkogen und ein abl-Onkogen exprimiert und in der Lage ist, mit einem aktivierten B-Lymphozyten zu fusionieren, worin die Fusion des Kaninchen-Fusionspartners und des aktivierten B-Lymphozyten in einem Hybridom resultiert, das ein Immunglobulin produziert.

2. Kaninchen-Fusionspartner nach Anspruch 1, der der Kaninchen-Fusionspartner der ATCC-Zugriffsnummer HB-11870 ist.

3. Kaninchen-Hybridomzelllinie, die aus der Fusion eines Kaninchen-Fusionspartners nach Anspruch 1 oder Anspruch 2 und eines aktivierten B-Lymphozyten resultiert.

4. Kaninchen-Hybridomzelllinie nach Anspruch 3, die die Hybridomzelllinie der ATCC-Zugriffsnummer HB-11871 ist.

5. Kaninchen-Plasmazytomzelllinie der ATCC-Zugriffsnummer CRL-11872.

6. Befruchtete Kanincheneizelle, umfassend
(i) eine Expressionskassette, die ein myc-Onkogen enthält, und eine Expressionskassette, die ein abl-Onkogen enthält, oder
(ii) eine Expressionskassette, die ein myc-Onkogen und ein abl-Onkogen enthält, worin ein transgenes Kaninchen, das sich aus dieser Eizelle entwickelt, Keimzellen und Somazellen umfasst, die diese zwei Onkogene enthalten, worin die Expression dieser zwei Onkogene zusammen in seinen B-Lymphozyten zur Transformation dieser B-Lymphozyten führt.

7. Transgenes Kaninchen, dessen Keimzellen und Somazellen ein myc-Onkogen und ein abl-Onkogen umfassen, die jeweils operabel an eine Transkriptionsstartregion gebunden sind, die für Expression in Kaninchen-B-Lymphozyten spezifisch ist, worin die Expression dieser zwei Onkogene zusammen zur Transformation dieser B-Lymphozyten führt.

8. Kaninchen-Fusionspartner nach Anspruch 1, befruchtete Kanincheneizelle nach Anspruch 6 oder transgenes Kaninchen nach Anspruch 7, worin jedes dieser Onkogene die Wildtyp-Transkriptionsstartregion für das Onkogen und einen Lymphozyten-spezifischen Enhancer umfasst.

9. Kaninchen-Fusionspartner, befruchtete Kanincheneizelle oder transgenes Kaninchen nach Anspruch 8, worin die Onkogene ein c-myc-Onkogen, das operabel an Immunglobulin-Leichtketten-Enhancer gebunden ist, und ein v-abl-Onkogen, das operabel an einen Immunglobulin-Leicht- oder -Schwerketten-Enhancer gebunden ist, sind.

10. Kaninchen-Fusionspartner, befruchtete Kanincheneizelle oder transgenes Kaninchen nach Anspruch 9, worin die Onkogene ein E_{κ}-myc-Onkogen und ein E_{µ}-abl-Onkogen sind.

11. Verfahren zur Herstellung eines Kaninchen-Fusionspartners, umfassend:
(a) Kultivieren von transformierten B-Lymphozyten, die von einem transgenen Kaninchen nach Anspruch 7 stammen, in Gegenwart eines Selektionsmittels; und
(b) Selektieren dieser transformierten B-Lymphozyten, worin die Selektion zu B-Lymphozyten führt, die ein selektierbares Merkmal umfassen und zur Verwendung als Kaninchen-Fusionspartner geeignet sind.

12. Verfahren nach Anspruch 11, worin die Selektion zu HGPRT-Zellen führt.

13. Verfahren zur Herstellung einer Kaninchen-Hybridomzelllinie, umfassend das Fusionieren des Kaninchen-Fusionspartners nach Anspruch 1 oder Anspruch 2 mit einem aktivierten Kaninchen-B-Lymphozyten unter Fusionsbedingungen und selektiven Bedingungen, um ein Hybridom zu erhalten, das in der Lage ist, Immunglobuline zu produzieren.

14. Verfahren zur Herstellung von monoklonalen Kaninchen-Immunglobulinen, umfassend das Herstellen einer Kaninchen-Hybridomzelllinie durch das Verfahren nach Anspruch 13 und das Kultivieren dieses Hybridoms unter solchen Bedingungen, dass die Immunglobuline produziert werden.

15. Verfahren nach Anspruch 14, worin der aktivierte Kaninchen-B-Lymphozyt aus einem Kaninchen, dem ein Antigen injiziert wurde, isoliert wird.

16. Verfahren zum Kultivieren eines Kaninchen-Fusionspartners, umfassend das Züchten der Zellen eines Kaninchen-Fusionspartners nach Anspruch 1 oder Anspruch 2 in einem angereicherten Kulturmedium, das Aminosäuren, essentielle Aminosäuren, Pyruvat und Glutamin umfasst.

## Revendications

1. Partenaire de fusion de lapin comprenant un lymphocyte B de lapin transformé qui exprime un oncogène myc et un oncogène abl et est capable de fusion avec un lymphocyte B activé, où la fusion du partenaire de fusion de lapin et du lymphocyte B activé a pour résultat un hybridome qui produit une immunoglobuline.

2. Partenaire de fusion de lapin selon la revendication 1, qui est le partenaire de fusion de lapin du Numéro d'Accession ATCC HB-11870.

3. Lignée de cellules d'hybridome de lapin qui résulte de la fusion d'un partenaire de fusion de lapin de la revendication 1 ou de la revendication 2 et d'un lymphocyte B activé.

4. Lignée de cellules d'hybridome de lapin de la revendication 3 qui est la lignée de cellules hybridomes du Numéro d'Accession ATCC HB-11871.

5. Lignée de cellules de plasmacytome de lapin du Numéro d'Accession ATCC CRL-11872.

6. OEuf de lapin fertilisé comprenant
(i) une cassette d'expression contenant un oncogène myc et une cassette d'expression contenant un oncogène abl, ou
(ii) une cassette d'expression contenant un oncogène myc et un oncogène abl, où un lapin transgénique développé à partir dudit oeuf comprend des cellules germinales et des cellules somatiques qui contiennent lesdits deux oncogènes, où l'expression desdits oncogènes ensemble dans ses lymphocytes B a pour résultat de la transformation desdits lymphocytes B.

7. Lapin transgénique dont les cellules germinales et les cellules somatiques comprennent un oncogène myc et un oncogène abl, chacun opérativement enchaîné à une région d'initiation de transcription spécifique de l'expression dans les lymphocytes B de lapin, où l'expression desdits deux oncogènes ensemble a pour résultat la transformation desdits lymphocytes B.

8. Partenaire de fusion de lapin de la revendication 1, l'oeuf de lapin fertilisé de la revendication 6, ou le lapin transgénique de la revendication 7, où chaque oncogène comprend la région d'initiation de transcription du type sauvage pour ledit oncogène et un enrichisseur spécifique des lymphocytes.

9. Partenaire de fusion de lapin, oeuf de lapin fertilisé ou lapin transgénique de la revendication 8, où lesdits oncogènes sont un oncogène du type c-myc opérativement enchaîné à un enrichisseur de chaîne légère d'immunoglobuline; et un oncogène v-abl opérativement enchaîné à un enrichisseur de chaîne lourde ou légère d'immunoglobuline.

10. Partenaire de fusion de lapin, oeuf de lapin fertilisé ou lapin transgénique de la revendication 9, où lesdits oncogènes sont un oncogène Eₖ-myc et un oncogène E_{µ}-abl.

11. Méthode de préparation d'un partenaire de fusion de lapin comprenant:
(a) la culture de lymphocytes B transformés obtenus d'un lapin transgénique selon la revendication 7 en présence d'un agent de sélection; et
(b) la sélection desdits lymphocytes B transformés où ladite sélection a pour résultat des lymphocytes B qui comprennent un trait sélectionnable et sont appropriés pour une utilisation en tant que partenaire de fusion de lapin.

12. Méthode de la revendication 11, où ladite sélection a pour résultat des cellules HGPRT.

13. Méthode de production d'une lignée de cellules d'hybridome de lapin comprenant la fusion du partenaire de fusion de lapin de la revendication 1 ou de la revendication 2 avec un lymphocyte B de lapin activé en conditions de fusion et sélectives pour obtenir un hybridome qui est capable de produire des immunoglobulines.

14. Méthode de production d'immunoglobulines monoclonales de lapin comprenant la production d'une lignée de cellules d'hybridome de lapin par la méthode de la revendication 13 et la culture dudit hybridome en conditions telle que lesdites immunoglobulines soient produites.

15. Méthode de la revendication 14, où ledit lymphocyte B de lapin activé est isolé d'un lapin ayant reçu une injection d'un antigène.

16. Méthode de culture d'un partenaire de fusion de lapin qui comprend la croissance des cellules d'un partenaire de fusion de lapin de la revendication 1 ou de la revendication 2 dans un milieu de culture enrichi comprenant des acides aminés, des acides aminés essentiels, du pyruvate et de la glutamine.
